# EUROPEAN PATENT APPLICATION

(11) **EP 3 178 946 A2**
(43) Date of publication of application: **14.06.2017**
(21) Application number: 16202429.3
(22) Date of filing: 06.12.2016
(51) Int. Cl.: C12R 1/02, C12N 15/63, C12N 15/74

(54) **ISOLATED POLYNUCLEOTIDE INCLUDING PROMOTER REGION, HOST CELL INCLUDING THE SAME, AND METHOD OF EXPRESSING TARGET GENE USING THE HOST CELL**

(30) Priority: 09.12.2015 KR 20150175342
(71) Applicant: Samsung Electronics Co., Ltd., Gyeonggi-do 16677 (KR)
(72) Inventor: YUN, Jiae, Suwon-si Gyeonggi-do 16678 (KR); RHEE, Hongsoon, Suwon-si Gyeonggi-do 16678 (KR)
(74) Representative: Grünecker Patent- und Rechtsanwälte PartG mbB

(57) **Abstract**

Provided is a recombinant polynucleotide including a promoter region derived from an acetic acid-producing bacterium and a polynucleotide sequence encoding a target protein operably linked to the promoter, a host cell including the same, and a method of expressing a target gene or protein using the host cell.

## Description

### INCORPORATION-BY-REFERENCE OF MATERIAL SUBMITTED ELECTRONICALLY

Incorporated by reference in its entirety herein is a computer-readable nucleotide/amino acid sequence listing submitted concurrently herewith and identified as follows: One 12,193 Byte ASCII (Text) file named "8.SI50641US_Sequence List.TXT," created on July 14, 2016.

### BACKGROUND

### 1. Field

The present disclosure relates to an isolated recombinant polynucleotide including a promoter region derived from an acetic acid-producing bacterium and a nucleotide sequence encoding a target protein operably linked to the promoter, a host cell including the same, and a method of expressing a target gene using the host cell.

### 2. Description of the Related Art

A promoter is required for expression of a target gene in a microorganism. The promoter is a region of DNA where an RNA polymerase binds during the transcription of DNA into mRNA. The binding of RNA polymerase, or other transcription factors, to the promoter region and the subsequent level of mRNA expression is dependent upon the DNA base sequence and the length of the promoter. In other words, the promoter determines the expression level of the gene under given conditions. Therefore, to improve microorganisms as desired, a proper promoter capable of expressing a target gene at a desired expression level is needed.

Acetic acid bacteria generally refer to bacteria that produce acetic acid during fermentation for vinegar production. Acetic acid bacteria have the ability to produce many organic acids such as acetic acid, and thus, have been traditionally used in the food and beverage industry. Additionally, it was revealed that a vinegar film produced by the acetic acid bacteria during fermentation for vinegar production is composed of cellulose. Such microbial cellulose is highly sought after as it has many industrial applications. However, it is difficult to produce large quantities of the microbial cellulose due to a low production rate in acetic acid bacteria. Therefore, it is crucial to increase the production rate of microbial cellulose in acetic acid bacteria. This invention provides such a method and host cell to increase the production rate of microbial cellulose in acetic acid bacteria.

### SUMMARY

One aspect of the invention provides a recombinant polynucleotide comprising a promoter region having a nucleotide sequence comprising positions 421 to 450 of SEQ ID NO: 1, a nucleotide sequence comprising positions 248 to 273 of SEQ ID NO: 2, or a nucleotide sequence comprising SEQ ID NO: 3, and a nucleotide sequence encoding a target protein operably linked to the promoter region.

Another aspect provides a host cell including the recombinant polynucleotide.

Still another aspect provides a method of expressing a target gene to produce a target protein using the host cell.

### BRIEF DESCRIPTION OF THE DRAWINGS

These and/or other aspects will become apparent and more readily appreciated from the following description of the exemplary embodiments, taken in conjunction with the accompanying drawing in which:

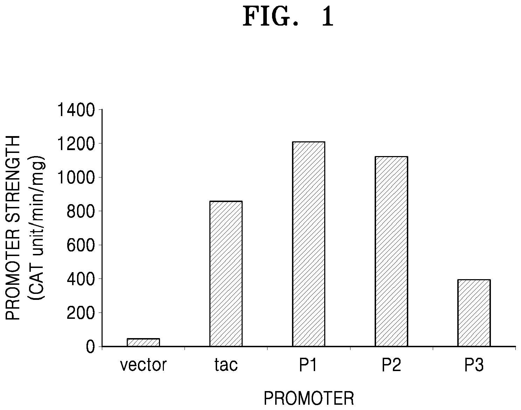

FIG. 1 shows results of a chloramphenicol acetyltransferase (CAT) assay.

### DETAILED DESCRIPTION

One aspect of the invention provides a recombinant polynucleotide including a promoter region having a nucleotide sequence of positions 421 to 450 of SEQ ID NO: 1, a nucleotide sequence of positions 248 to 273 of SEQ ID NO: 2, or a nucleotide sequence of SEQ ID NO: 3, and a target gene sequence encoding a target protein.

The term "promoter", as used herein, refers to a DNA region to which an RNA polymerase binds to initiate transcription of a gene operably linked thereto. The sequence of a promoter may be modified (e.g., mutated) by those skilled in the art, such that the resulting promoter has the same or similar activity. Thus, a promoter having a sequence homology of, for example, 70% or higher, 80% or higher, 90% or higher, or 95% or higher to the nucleotide sequence of positions 421 to 450 of SEQ ID NO: 1, the nucleotide sequence of positions 248 to 273 of SEQ ID NO: 2, or the nucleotide sequence of SEQ ID NO: 3 are included in the scope of the invention.

Additionally, a fragment including sequences having the promoter activity in the nucleotide sequence of positions 421 to 450 of SEQ ID NO: 1, the nucleotide sequence of positions 248 to 273 of SEQ ID NO: 2, or the nucleotide sequence of SEQ ID NO: 3, for example, sequences (hereinafter, referred to as "variant") of a predicted transcription start site and -10 element may be also included in the scope of the present invention.

The promoter may comprise, consist essentially of, or consist of a nucleotide sequence of SEQ ID NO: 1, 2, or 3.

The recombinant polynucleotide may be a vector. In the recombinant polynucleotide, the target gene may be operably linked to the promoter region. The target gene may be operably linked downstream of the promoter region. The term "operably linked" as used herein means that a gene to be expressed is functionally linked to its control sequences so that expression of the gene is allowed. The vector may further include a replication origin, a promoter control site, a ribosome binding site, a transcription termination site, a selection marker, or a combination thereof, as well as the promoter or variant thereof and the target gene.

The term "vector", is a term known to those of ordinary skill in the art and refers to a construct for transferring a nucleic acid into cells. The vector may include, for example, a plasmid or a vector derived from a virus. A "plasmid" refers to a circular, double-stranded DNA loop. Certain vectors are capable of autonomous replication in a host cell into which they are introduced (e.g., bacterial vectors having a bacterial origin of replication). Other vectors are integrated into the genome of a host cell upon introduction into the host cell, thereby being replicated along with the host genome. Moreover, certain vectors may direct expression of genes to which they are operatively linked. Such vectors are referred to herein as "expression vectors". In general, expression vectors of utility in recombinant DNA techniques are often in the form of plasmids. The vector used herein may include, for example, a plasmid expression vector, a viral expression vector, and a viral vector capable of performing a function equivalent thereto.

The vectors may include one or more regulatory sequences, selected on the basis of the host cells to be used for expression, and are operatively linked to the nucleic acid sequence to be expressed. "Operatively linked" means that the nucleotide sequence of interest is linked to the regulatory sequence(s) in a manner which allows for expression of the nucleotide sequence in the host cell. The term "regulatory sequence" is intended to include promoters, enhancers and other expression control elements. Regulatory sequences include those which direct constitutive expression of a target nucleic acid in many types of host cells and those which direct expression of the target nucleic acid only in particular host cells (e.g., tissue-specific regulatory sequences). It will be appreciated by those skilled in the art that the design of the expression vector may depend upon factors such as choice of the host cell to be transformed, an expression level of a protein desired, or the like. The expression vector of the present invention may be introduced into the host cell to express the protein.

The plasmid may be a bacterial cloning vector. These cloning vectors may include a site that allows DNA fragments to be inserted, for example, a multiple cloning site or a polylinker having several commonly used restriction sites to which DNA fragments may be ligated. After the gene of interest is inserted, the plasmids are introduced into bacteria by transformation. These plasmids may include a selectable marker, usually, an antibiotic resistance gene, which confer on the bacteria an ability to survive and proliferate in a selective growth medium containing the particular antibiotics. The cells after transformation are exposed to the selective media, and only cells containing the plasmid may survive. In this way, the antibiotics act as a filter to select only the bacteria containing the plasmid DNA. The vector may also contain other marker genes or reporter genes to facilitate selection of plasmid with the cloned insert. Thereafter, bacteria containing the plasmid may be grown in large amounts, harvested, and then isolated using various methods of plasmid preparation. A plasmid cloning vector may be used to clone DNA fragments of about 15 kbp or shorter. The vector may be a commercially available vector, for example, a pBR322, pUC, or TOPO cloning vector.

The target gene may be any gene encoding a target protein heterologous (non-native) to the promoter. The target gene may encode a protein involved in cellulose production, for example, synthesis. The gene encoding the target product may be a gene encoding permease, glucose kinase (GLK), phosphoglucomutase (PGM), UDP-glucose pyrophosphorylase (UGP), or cellulose synthase (CS). The gene encoding the target product may be an *E*. *coli* (Ec) glcP gene, a *Zymomonas mobilis* (Zm) glk gene, a *Komagataeibacter xylinus* (Kx) pgm gene, an *E*. *coli* (Ec) galU gene, a *Xanthomonas campestris* (Xc) UGP gene, or a *Komagataeibacter xylinus* (Kx) bcsABCD gene.

The recombinant polynucleotide may be produced by any technique. The term "recombinant" in this respect means that the polynucleotide is produced by genetic engineering, and is non-naturally occurring. The recombinant polynucleotide maybe synthetic, or semisynthetic.

Under aerobic conditions, the recombinant polynucleotide may increase expression of the operably linked target gene by 0.5 time or higher, for example, 1 time, 1.2 times, 1.3 times, or 1.4 times or higher, compared to that of the gene operably linked to a tac promoter, based on mRNA or protein level.

Another aspect provides a host cell including the recombinant polynucleotide.

The host cell may be an acetic acid-producing bacterium. The host cell may be a cell of the family *Acetobacteraceae.* The cell of the family *Acetobacteraceae* may be a cell of the genus *Komagataibacter,* the genus *Gluconacetobacter,* the genus *Acetobacter,* or the genus *Gluconobacter.* The host cell may be a *Komagataibacter xylinus* (also called *"Acetobacter xylinum"*) cell. The host cell may be a recombinant host cell.

In the host cell, the recombinant polynucleotide may be a vector. In the polynucleotide, the target gene may be operably linked to the promoter region. The target gene may encode the target protein. The target gene may encode a protein involved in cellulose production, for example, cellulose synthesis. The gene encoding the target product may be a gene encoding permease, glucose kinase (GLK), phosphoglucomutase (PGM), UDP-glucose pyrophosphorylase (UGP), or cellulose synthase (CS). The gene encoding the target product may be an *E*. *coli* (Ec) glcP gene, a *Zymomonas mobilis* (Zm) glk gene, a *Komagataeibacter xylinus* (Kx) pgm gene, an *E*. *coli* (Ec) galU gene, a *Xanthomonas campestris* (Xc) UGP gene, or a *Komagataeibacter xylinus* (Kx) bcsABCD gene.

The vector may be introduced into the host cell to clone the target gene or to express the target gene. The introduction of the vector may be performed by applying appropriate standard techniques known in the art, depending on the host cell, for example, by electroporation, heat-shock, calcium phosphate (CaPO₄) precipitation, calcium chloride (CaCl₂) precipitation, microinjection, a polyethylene glycol (PEG) method, a DEAE-dextran method, a cationic liposome method, a lithium acetate-DMSO method, or a combination thereof.

In the vector introduced to the host cell, a gene encoding the target protein may be operably linked downstream of the promoter. The vector may further include a replication origin, a promoter control site, a ribosome binding site, a transcription termination site, a selection marker, or a combination thereof. The host cell may express the gene operably linked to the promoter, for example, under anaerobic conditions. For example, the gene may be highly expressed under aerobic conditions and may also maintain a relatively high level of gene expression under anaerobic conditions. Under aerobic conditions, an expression level of the gene may be 0.5 times or higher, for example, 1.0 times or higher, 1.2 times or higher, 1.3 times or higher, or 1.4 times or higher than the expression level of the same gene operably linked to a tac promoter, based on an mRNA or protein level.

Another aspect of the invention provides a method of expressing the target gene, the method including culturing the host cell including the recombinant polynucleotide including the promoter region comprising the nucleotide sequence of positions 421 to 450 of SEQ ID NO: 1, the nucleotide sequence of positions 248 to 273 of SEQ ID NO: 2, or the nucleotide sequence of SEQ ID NO: 3 and the target gene operably linked to the promoter region to express the target protein.
The method includes culturing the host cell so as to express the target gene and produce the target protein.

In the method, the host cell may be a cell of the family *Acetobacteraceae.* The target gene may encode a protein involved in cellulose production, for example, synthesis. The gene encoding the target product may be a gene encoding permease, glucose kinase (GLK), phosphoglucomutase (PGM), UDP-glucose pyrophosphorylase (UGP), or cellulose synthase (CS). The gene encoding the target product may be an *E*. *coli* (Ec) glcP gene, a *Zymomonas mobilis* (Zm) glk gene, a *Komagataeibacter xylinus* (Kx) pgm gene, an *E*. *coli* (Ec) galU gene, a *Xanthomonas campestris* (Xc) UGP gene, or a *Komagataeibacter xylinus* (Kx) bcsABCD gene.

The method may be, for example, a method of culturing the vector-introduced host cells to produce a final product in a biosynthetic pathway involving the protein encoded by the target gene. The target gene may be, for example, involved in production, for example, synthesis of a product selected from the group consisting of cellulose, L-amino acids, lactic acid, acetic acid, succinic acid, and combinations thereof. Therefore, the method may be used to produce the final product of the gene, for example, a product selected from the group consisting of cellulose, L-amino acids, lactic acid, acetic acid, succinic acid, and combinations thereof under aerobic or anaerobic conditions. In the method, the product may be, for example, produced under aerobic conditions, and maintained under anaerobic conditions. The host cell may be, for example, *K. xylinus* KCCM 41431 that is introduced with a vector, in which the gene involved in the production, for example, synthesis of the product is operably linked to the promoter having the nucleotide sequence of SEQ ID NO: 1, 2, or 3 or a variant thereof.

The culturing of the host cell may be performed according to general methods known in the art. A medium used for the culturing may include a sugar source, for example, sugar and carbohydrate (e.g., glucose, saccharose, lactose, fructose, maltose, and starch), oil and fat, (e.g., soybean oil, sunflower oil, castor oil, and coconut oil), a fatty acid, (e.g., palmitic acid, stearic acid, and linolenic acid), an alcohol, (e.g., glycerol and ethanol), and an organic acid, (e.g., acetic acid), alone or in a mixture. The medium may include as a nitrogen source, for example, peptone, yeast extract, meat extract, malt extract, corn steep liquor, soy meal and urea, or an inorganic compound, e.g., ammonium sulfate, ammonium chloride, ammonium phosphate, ammonium carbonate and ammonium nitrate, singly or in a mixture. The medium may include as a phosphorous source, for example, potassium dihydrogen phosphate, dipotassium hydrogen phosphate, or a corresponding sodium-containing salt thereof. The medium may include, for example, a metal salt, e.g., magnesium sulfate or iron sulfate, which is essential for growth. Also, in the culturing, substances essential for growth, such as amino acids and vitamins, or suitable precursors may be added to the culture. Those components may be added to the culture in a proper manner, for example, in a batch or continuous manner during the culturing.

The culturing may be performed under aerobic conditions.

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. As used herein, the term "and/or" includes any and all combinations of one or more of the associated listed items.

Hereinafter, the present invention will be described in more detail with reference to Examples. However, these Examples are for illustrative purposes only, and the scope of the invention is not intended to be limited by these Examples.

### Example 1: Exploration of promoters and Identification of P1, P2, and P3

In this Example, three kinds of promoters which may be utilized in gene overexpression were explored in acetic acid bacteria, especially, microbial cellulose-producing bacteria of the genus *Komagataibacter.* All of these promoters were derived from a genome of *Komagataibacter xylinus* KCCM 41431 which is a natural microbial cellulose-producing bacterium.

### (1) Exploration of promoter

The genomic DNA of *K. xylinus* KCCM 41431 was extracted, and partially digested with Sau3Al. Of digestion products, DNA fragments having a size of 0.5 to 1.5 kb were extracted from a 1% agarose gel. Each of the extracted DNA fragments was ligated to a promoter exploration vector, pTSaP having a nucleotide sequence of SEQ ID NO: 4, which was digested with BgIII. These vectors were transformed into *E*. *coli* Top10 strain, and then plated on Luria Delbruck (LB) solid media containing 10 µg/ml of tetracycline and 5 µg/ml of chloramphenicol. Plasmids were isolated from pooled colonies, and *K.xylinus* KCCM 41431 was transformed with each of the plasmid by electroporation, and then plated on a Hestrin-Schramm (HS: 20 g/l of glucose, 5 g/l of yeast extract, 5 g/l of polypeptone, 2.7 g/l of Na₂PO₄ and 1.15 g/l of citric acid) solid medium containing 5 µg/ml tetracycline to obtain colonies. In pTSaP, the inserted genomic DNA and a reporter gene, chloramphenicol acetyltransferase (*cat*) gene were operably linked. The promoter exploration vector pTSaP was a custom-made vector. In pTSaP, a replication origin pSa ori which allows initiation of replication in cells of the genus *Komagataibacter,* a tetracycline resistance gene, an *E*. *coli* replication origin pUC ori, and the reporter gene cat are operably linked to a transcription terminator.

*K.xylinus* KCCM 41431 colonies thus obtained were passaged in HS solid media containing 5 µg/ml of tetracycline and 120 µg/ml of chloramphenicol, respectively. Plasmids were isolated from *K.xylinus* KCCM 41431 colonies which were successfully passaged by culturing at 30°C for 48 hours or longer. Then, the plasmids were used as a template and polynucleotides of SEQ ID NOS: 5 and 6 were used as primers to perform sequencing.

As a result of the sequencing, a sequence of DNA cloned into the BgIII site of the pTSaP vector was revealed, and the obtained promoters were designated as P1, P2, and P3, respectively. The P1, P2, and P3 promoters have nucleotide sequences of SEQ ID NOS: 1, 2, and 3, respectively. Transcription start sites of these sequences were predicted by a promoter prediction program (Genome2D webserver for analysis and visualization of bacterial genomes and transcriptome data, de Jong et al. BMC Genomics 2012, 13: 299). As a result, the predicted promoter region of SEQ ID NO: 1 was a nucleotide sequence of positions 421 to 450, 'TATAATGCATTCTGATATTTTGTTGTTAT' (SEQ ID NO: 8), and the transcription start site was 'T' at position 455. The predicted promoter region of SEQ ID NO: 2 was a nucleotide sequence of positions 248 to 273, 'TTAAATTTTTCATACTTATTAATGTAAAAT' (SEQ ID NO: 9), and the transcription start site was 'T' at position 283.

### (2) Evaluation of promoter strength

The promoter activity of the inserted genomic DNA was determined by measuring expression strength of the reporter gene, *cat,* i.e., strength of CAT activity. With regard to control groups, pTSaP introduced without the genomic DNA was used as a negative control group, and pTSaP containing a generally used *tac* promoter (SEQ ID NO: 7) was used as a positive control group.

CAT activity was determined by a CAT assay that measures acetylated chloramphenicol, and the CAT assay was performed as follows. Acetyl-CoA was reacted with chloramphenicol in the presence of CAT enzyme and 5,5'-dithio-bis (2-nitrobenzoic acid (DTNB) to produce acetyl-chloramphenicol and CoA. In this regard, CoA was reacted with DTNB to be converted into 5-thio-2-nitrobenzoate (TNB) having absorbance at 412 nm. That is, *K.xylinus* colonies obtained in section (1) and control groups were cultured in HS liquid media containing tetracycline (5 µg/ml) and cellulose (0.5%, Sigma C2730) at 30°C for 24 hours under stirring at 220 rpm. The bacteria were harvested and suspended in PBS buffer, and then disrupted by sonication, followed by centrifugation. A supernatant was collected and a crude protein was obtained. Next, 10 µg of the crude protein per 1 ml of a reaction solution was mixed and reacted with acetyl-CoA (200 µg/mL), chloramphenicol (100 µg/mL), and DTNB (50 mg/mL), and absorbance at 412 nm was measured over time. The measured absorbance was applied to the following equation to calculate the activity.

activity (units/ml enzyme)=(Δ412 nm/min test-Δ412 nm/min Blank)(df)/(0.0136) (df: dilution factor)

FIG. 1 shows the result of CAT assay. In FIG. 1, vector (control group), tac, P1, P2, and P3 on the horizontal axis represent the pTSaP vector introduced without the genomic DNA, the pTSaP vector introduced with the tac promoter, and the pTSaP vector introduced with the promoter of SEQ ID NO: 1, 2, or 3, respectively. As shown in FIG. 1, P1, P2, and P3 showed strength 1.41 times, 1.31 times, and 0.46 times higher than that of the positive control group, tac promoter, respectively. P1, P2, and P3 also showed marked expression-improving effects, compared to the negative control group.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. An isolated recombinant polynucleotide comprising
(i) a promoter region having a nucleotide sequence comprising positions 421 to 450 of SEQ ID NO: 1, a nucleotide sequence comprising positions 248 to 273 of SEQ ID NO: 2, or a nucleotide sequence comprising SEQ ID NO: 3; and
(ii) a nucleotide sequence encoding a target protein operably linked to the promoter region.

2. The polynucleotide of claim 1, wherein the promoter comprises SEQ ID NO: 1, 2, or 3.

3. The polynucleotide of claim 1 or 2, wherein the polynucleotide is a vector.

4. The polynucleotide of any one of claims 1-3, wherein the target protein is a protein involved in cellulose production.

5. The polynucleotide of claim 4, wherein the target protein is selected from the group consisting of permease, glucose kinase (GLK), phosphoglucomutase (PGM), UDP-glucose pyrophosphorylase (UGP), and cellulose synthase (CS).

6. A host cell comprising the recombinant polynucleotide of any one of claims 1-5.

7. The host cell of claim 6, wherein the host cell is an acetic acid-producing bacterium.

8. The host cell of claim 6 or 7 wherein the host cell is a cell of the family *Acetobacteraceae.*

9. The host cell of claim 8, wherein the cell of the family *Acetobacteraceae* is a cell of the genus *Komagataibacter,* the genus *gluconacetobacter,* the genus *acetobacter,* or the genus *gluconobacter.*

10. The host cell of any one of claims 6-9, wherein the host cell is a *Komagataibacter xylinus* cell.

11. A method of producing a target protein, the method comprising culturing a host cell comprising a recombinant polynucleotide of any one of claims 1-5 to express the target protein.

12. The method of claim 11, wherein the host cell is a cell of the family *Acetobacteraceae.*

13. The method of claim 11 or 12, wherein the target gene encodes a protein involved in cellulose production.

14. The method of claim 13, wherein the target protein is selected from the group consisting of permease, glucose kinase (GLK), phosphoglucomutase (PGM), UDP-glucose pyrophosphorylase (UGP), and cellulose synthase (CS).

15. The method of any one of claims 11-14, wherein the culturing is performed under aerobic conditions.


<110>     Samsung Electronics Co., Ltd.

<120>     Isolated polynucleotide comprising promoter region, host cell
          comprising the same and Method of expressing a target gene using
          the host cell

<130>     EP109232FZSEpau

<140>     not yet assigned
<141>     herewith

<150>     KR10-2015-0175342
<151>     2015-12-09

<160>     9

<170>     KopatentIn 2.0

<210>     1
<211>     1141
<212>     DNA
<213>     Komagataibacter xylinus KCCM 41431

<400>     1
gatcgcgggc acgcgctgca ttgaccgcca gcgcggcggc agccgttttg gcgcccaccg        60

caccggactt gcgctggctg accgggtgag cgggcgcgca gccgccctgt ccagcacggg       120

gcagcagaag gcgctgctgc tgggcatcgt gctgtcgcat gcgcgcatac tcgcggcatg       180

ccgggggcag gtgcccatgc tcctgctcga tgagccgctg gtgcatctgg acgaagcccg       240

caggcaggcc ctgttccgcg ccgtgggccg catgcgcacg ggcgtgttcc tgaccggaac       300

ggacgccgaa cagttcgccc ccctgcgggg ccatgccgca ttcgaggcgc caggggcggg       360

taatcttgcc catgaggcct gatttgcgtg gggaatgctg gttccgacgg gcggttgggg       420

ctataatgca ttctgatatt ttgttgttat ccgctgtgga gcatctgccg gcatgtccga       480

tcaatccagt cccgatcaga aacacgatgc cgaggccaag ggcacagtag cgcccgcgcc       540

tgattatgat gaggcatcca tctcggtgct gcgggggctg gatgcggtgc gcaagcgccc       600

cggcatgtat attggcgata ccgatgacgg ctcgggcctg caccacatgg ccttcgaaat       660

cattgataac gcggtggatg aggcgcaggc cggtttcgcc accggctgcg tcgtcaccct       720

caatggcgat ggcagcgtga ccgtgcgcga tgacgggcgc ggcattccca ccggcatgca       780

ccatgaggaa ggggtgagtg cggcggaagt cgtgctgacc aagctgcatg cgggcggcaa       840

gttcaaccag aattcctaca aggtttccgg tggcctgcat ggcgtgggcg ctgcggtggt       900

caatgctttg tccgaatgga tggaagtgcg catctggcgc gatggcaagg agcatgtgat       960

ccgctttcag ggcggcgagc gtgatgaggc gctgcgcgtg gtgggcgaaa gtgctgagcc      1020

gcgcggcacg caggtcacat tcaagcccag tgccaagacc ttcgccaagg tggagttcga      1080

gttcccgatt ctcgagcgcc gcctgcgcga actggccttc ctcaattccg ggctcaggat      1140

c                                                                      1141

<210>    2
<211>    1058
<212>    DNA
<213>    Komagataibacter xylinus KCCM 41431

<400>    2
gatcggtcca cgtatcaata aactgtgcgg actggtagag ggccatcccg ttctccattg    60

tttttcatcg gctggctggg ctgttaaata acctttagca tgcatcttat gtggcggcac    120

atccatcccc catgcagcat gctataatcc tgtgcttttt ccccacctgt gggtattgct    180

tcccatgcgg ggcaggtaga ttatttatga gggcatctgt cacaagccaa catcttttca    240

gtacagaatt aaatttttca tacttattaa tgtaaaatgt aatttatatt cctgttttat    300

ccatattgaa attattggat gtagaaaata acaaatttat aaatagcata acagggttcg    360

ttttatagga aaatattcat tgaaacgttt tgcgaaaata acgtaacgat taaaaaacaa    420

taaaagtttt ttcaatgcag ctttgtcaaa aaaaacttat cggatactgg cagttgattg    480

aaggggttgt tcacaaaacc cgccattatc ccgtttcctg cctgcctttg gcgtgccggg    540

tagcatgatg cggacagcat gtaactgaaa ggactgtctt tcagtctgga gaacgaagcg    600

tcagcgactg gtcaactgtt tgtgtcacaa tggcattcag cgcgccatgt gctgctgtcc    660

attgaaaggg atcggcatcg ttattgaagg tcgggtcaag ctggtcgccc cgtaccgtgc    720

cataggcctg aaaatacatg tcgggccggg cggggatggt gtccatcgtc catgtccagt    780

catcggacag gagagctgta gcgcctacct gcgcgcaggg gcgcgtgggt gacagggtca    840

cggctgccgt cgtcatgagg gctgcctgcg ggcggtggta gcggctgtct ccgccaatat    900

cttccatctg caggtcgcgg cggcgcgctt cctcggcggg cgggatgttg cgccagccat    960

cgccatcggg gcctgcgtgg gcgcggtcga gcgggtccga tgcagggcca aaggcgtggc    1020

ctgcgtaatt gagcgcggtg cctgcgggca catggatc    1058

<210>    3
<211>    535
<212>    DNA
<213>    Komagataibacter xylinus KCCM 41431

<400>    3
aacttcggcg gcgcccgagc gtgaacagca cgggctgacc aacctgtgcg cgcgcggcgg    60

ctacgtcctg gcggaagccg aagggacgcg gcaggtcacg ctggtcgcca cggggcacga    120

ggcgatactg gcgctggcgg cacgcaaact gttgaaggac gcaggggttg cggcggctgt    180

cgtatccctt ccatgctggg aactgttcgc cgcgcaaaaa atgacgtatc gtgccgccgt    240

gctgggaacg gcaccccgga tcggcattga agccgcgtca gggtttggat gggaacgctg    300

gcttgggaca gacgggctgt ttgttggcat tgacgggttc gggacggccg ccccggacca    360

gccggacagc gcgactgaca tcacgccgga acggatctgc cgcgacgcgc tgcgtctggt 420

ccgtcccctg tccgataccc tgactgaacc ggcgggagga aacggcgcgc cgcccgggat 480

gacatcggcc gatgtcagtg tgtgaaatgt cagaccttac ggagaaaata agaaa 535

<210> 4
<211> 4843
<212> DNA
<213> Artificial Sequence

<220>
<223> pTSaP: promoter screening vector

<400> 4
aagaacatgt gagcaaaagg ccagcaaaag gccaggaacc gtaaaaaggc cgcgttgctg 60

gcgtttttcc ataggctccg cccccctgac gagcatcaca aaaatcgacg ctcaagtcag 120

aggtggcgaa acccgacagg actataaaga taccaggcgt ttccccctgg aagctccctc 180

gtgcgctctc ctgttccgac cctgccgctt accggatacc tgtccgcctt tctcccttcg 240

ggaagcgtgg cgctttctca tagctcacgc tgtaggtatc tcagttcggt gtaggtcgtt 300

cgctccaagc tgggctgtgt gcacgaaccc cccgttcagc ccgaccgctg cgccttatcc 360

ggtaactatc gtcttgagtc caacccggta agacacgact tatcgccact ggcagcagcc 420

actggtaaca ggattagcag agcgaggtat gtaggcggtg ctacagagtt cttgaagtgg 480

tggcctaact acggctacac tagaagaaca gcatttggta tctgcgctct gctgaagcca 540

gttaccttcg gaaaaagagt tggtagctct tgatccggca aacaaaccac cgctggtagc 600

ggtggttttt ttgtttgcaa gcagcagatt acgcgcagaa aaaaaggatc tcaagaagat 660

cctttgatct tttctacggg gtctgacgct cagtggaacg aaaactcacg ttaattctca 720

tgtttgacag cttatcatcg ataagcttta atgcggtagt ttatcacagt taaattgcta 780

acgcagtcag gcaccgtgta tgaaatctaa caatgcgctc atcgtcatcc tcggcaccgt 840

caccctggat gctgtaggca taggcttggt tatgccggta ctgccgggcc tcttgcggga 900

tatcgtccat tccgacagca tcgccagtca ctatggcgtg ctgctagcgc tatatgcgtt 960

gatgcaattt ctatgcgcac ccgttctcgg agcactgtcc gaccgctttg gccgccgccc 1020

agtcctgctc gcttcgctac ttggagccac tatcgactac gcgatcatgg cgaccacacc 1080

cgtcctgtgg atcctctacg ccggacgcat cgtggccggc atcaccggcg ccacaggtgc 1140

ggttgctggc gcctatatcg ccgacatcac cgatggggaa gatcgggctc gccacttcgg 1200

gctcatgagc gcttgtttcg gcgtgggtat ggtggcaggc cccgtggccg ggggactgtt 1260

gggcgccatc tccttgcatg caccattcct tgcggcggcg gtgctcaacg gcctcaacct 1320

actactgggc tgcttcctaa tgcaggagtc gcataaggga gagcgtcgac cgatgccctt 1380

gagagccttc aacccagtca gctccttccg gtgggcgcgg ggcatgacta tcgtcgccgc 1440

acttatgact gtcttcttta tcatgcaact cgtaggacag gtgccggcag cgctctgggt 1500

cattttcggc gaggaccgct ttcgctggag cgcgacgatg atcggcctgt cgcttgcggt 1560

attcggaatc ttgcacgccc tcgctcaagc cttcgtcact ggtcccgcca ccaaacgttt 1620

cggcgagaag caggccatta tcgccggcat ggcggccgac gcgctgggct acgtcttgct 1680

ggcgttcgcg acgcgaggct ggatggcctt ccccattatg attcttctcg cttccggcgg 1740

catcgggatg cccgcgttgc aggccatgct gtccaggcag gtagatgacg accatcaggg 1800

acagcttcaa ggatcgctcg cggctcttac cagcctaact tcgatcactg gaccgctgat 1860

cgtcacggcg atttatgccg cctcggcgag cacatggaac gggttggcat ggattgtagg 1920

cgccgcccta taccttgtct gcctccccgc gttgcgtcgc ggtgcatgga gccgggccac 1980

ctcgacctga atggaagccg gcggcacctc gctaacggat tcaccactcc aagaattgga 2040

gccaattttt aaggcagtta ttggtgccct taaacgcctg gttgctacgc ctgaataagt 2100

gataataagc ggatgaatgg cagaaattcg aattcagcca gcaagacagc gatagagggt 2160

agttatccac gtgaaaccgc taatgccccg caaagccttg attcacgggg ctttccggcc 2220

cgctccaaaa actatccacg tgaaatcgct aatcagggta cgtgaaatcg ctaatcggag 2280

tacgtgaaat cgctaataag gtcacgtgaa atcgctaatc aaaaaggcac gtgagaacgc 2340

taatagccct ttcagatcaa cagcttgcaa acacccctcg ctccggcaag tagttacagc 2400

aagtagtatg ttcaattagc ttttcaatta tgaatatata tatcaattat tggtcgccct 2460

tggcttgtgg acaatgcgct acgcgcaccg gctccgcccg tggacaaccg caagcggttg 2520

cccaccgtcg agcgccagcg cctttgccca caacccggcg gccggccgca acagatcgtt 2580

ttataaattt ttttttttga aaaagaaaaa gcccgaaagg cggcaacctc tcgggcttct 2640

ggatttccga tcacctgtaa agtgggacca catgctgaac tccctatcac tgcatgagta 2700

gggaactgcc aggcatcaaa taaaacgaaa ggctcagtcg aaagactggg cctttcgttt 2760

tatctgttgt ttgtcggtga acgctctcct gagtaggaca aatccgccgg gagcggattt 2820

gaacgttgcg aagcaacggc ccggagggtg gcgggcagga cgcccgccat aaactgccag 2880

gcatcaaatt aagcagaagg ccatcctgac ggatggcctt tttgcgtttc tacaaactct 2940

gcaggtcgac tctagagatc taacttcggc ggcgcccgag cgtgaacagc acgggctgac 3000

caacctgtgc gcgcgcggcg gctacgtcct ggcggaagcc gaagggacgc ggcaggtcac 3060

gctggtcgcc acggggcacg aggcgatact ggcgctggcg gcacgcaaac tgttgaagga 3120

cgcaggggtt gcggcggctg tcgtatccct tccatgctgg gaactgttcg ccgcgcaaaa 3180

aatgacgtat cgtgccgccg tgctgggaac ggcaccccgg atcggcattg aagccgcgtc 3240

agggtttgga tgggaacgct ggcttgggac agacggcctg tttgttggca ttgacgggtt 3300

cgggacggcc gccccggacc agccggacag cgcgactgac atcacgccgg aacggatctg 3360

ccgcgacgcg ctgcgtctgg tccgtcccct gtccgatacc ctgactgaac cggcgggagg 3420

aaacggcgcg ccgcccggga tgacatcggc cgatgtcagt gtgtgaagat ctcccgggta 3480

ccgagctctc tagaaagaag gagggacgag ctattgatgg agaaaaaaat cactggatat 3540

accaccgttg atatatccca atggcatcgt aaagaacatt ttgaggcatt tcagtcagtt 3600

gctcaatgta cctataacca gaccgttcag ctggatatta cggccttttt aaagaccgta 3660

aagaaaaata agcacaagtt ttatccggcc tttattcaca ttcttgcccg cctgatgaat 3720

gctcatccgg aattccgtat ggcaatgaaa gacggtgagc tggtgatatg ggatagtgtt 3780

cacccttgtt acaccgtttt ccatgagcaa actgaaacgt tttcatcgct ctggagtgaa 3840

taccacgacg atttccggca gtttctacac atatattcgc aagatgtggc gtgttacggt 3900

gaaaacctgg cctatttccc taaagggttt attgagaata tgtttttcgt ctcagccaat 3960

ccctgggtga gtttcaccag ttttgattta aacgtggcca atatggacaa cttcttcgcc 4020

cccgttttca ccatgggcaa atattatacg caaggcgaca aggtgctgat gccgctggcg 4080

attcaggttc atcatgccgt ttgtgatggc ttccatgtcg gcagaatgct taatgaatta 4140

caacagtact gcgatgagtg gcagggcggg gcgtaatttt tttaaggcag ttattggtgc 4200

ccttaaacgc ctggttgcta cgcctgaata agtgataata agcggatgaa tggcagaaat 4260

tcgtcgaggc ggcacctcgc taacggattc accactccaa gaattggagc caatcaattc 4320

ttgcggagaa ctgtgaatgc gcaaaccaac ccttggcaga acatatccat cgcgtccgcc 4380

atctccagca gccgcacgcg gcgcatctcg gctgttttgg cggatgagag aagattttca 4440

gcctgataca gattaaatca gaacgcagaa gcggtctgat aaaacagaat ttgcctggcg 4500

gcagtagcgc ggtggtccca cctgacccca tgccgaactc agaagtgaaa cgccgtagcg 4560

ccgatggtag tgtggggtct ccccatgcga gagtagggaa ctgccaggca tcaaataaaa 4620

cgaaaggctc agtcgaaaga ctgggccttt cgttttatct gttgtttgtc ggtgaacgct 4680

ctcctgagta ggacaaatcc gccgggagcg gatttgaacg ttgcgaagca acggcccgga 4740

gggtggcggg caggacgccc gccataaact gccaggcatc aaattaagca gaaggccatc 4800

ctgacggatg gcctttttgc gtttctacaa actcttcctg tcg 4843

<210> 5
<211> 24
<212> DNA
<213> Artificial Sequence

<220>
<223> SeqF primer

<400>    5
atgttcttta cgatgccatt ggga                                              24


<210>    6
<211>    22
<212>    DNA
<213>    Artificial Sequence

<220>
<223>    SeqR primer


<400>    6
tctcctgagt aggacaaatc cg                                                22


<210>    7
<211>    188
<212>    DNA
<213>    Artificial Sequence

<220>
<223>    tac promoter


<400>    7
ggctgtgcag gtcgtaaatc actgcataat tcgtgtcgct caaggcgcac tcccgttctg      60

gataatgttt tttgcgccga catcataacg gttctggcaa atattctgaa atgagctgtt     120

gacaattaat catcggctcg tataatgtgt ggaattgtga gcggataaca atttcacaca     180

ggaaacat                                                               188


<210>    8
<211>    29
<212>    DNA
<213>    Artificial Sequence

<220>
<223>    P1 promoter


<400>    8
tataatgcat tctgatattt tgttgttat                                         29


<210>    9
<211>    30
<212>    DNA
<213>    Artificial Sequence

<220>
<223>    P2 promoter


<400>    9
ttaaattttt catacttatt aatgtaaaat                                        30